# EUROPEAN PATENT APPLICATION

(11) **EP 4 846 952 A1**
(43) Date of publication of application: **02.12.2026**
(21) Application number: 24920688.9
(22) Date of filing: 14.10.2024
(51) Int. Cl.: A61B 17/22, A61F 2/86, A61F 2/89, A61F 2/88, A61F 2/95

(54) **MECHANICAL THROMBECTOMY APPARATUS**

(30) Priority: 26.01.2024 KR 20240012201; 24.04.2024 KR 20240054805
(71) Applicant: Nventric Corporation, Seoul 08376 (KR)
(72) Inventor: NGO, Don Quy, Los Angeles, California 90065 (US); MIN, Sungwoo, Fullerton, California 92835 (US); MIN, Jiyoung, Seoul 04739 (KR)
(74) Representative: Bringer IP
(86) International application number: PCT/KR2024/015452
(87) International publication number: WO 2025/159272

(57) **Abstract**

A mechanical thrombectomy device is provided. The mechanical thrombectomy device may include a support wire and a clot arrestor connected to the support wire and including an expandable frame. The expandable frame may include a proximal portion in which a first plurality of cells of the expandable frame are connected to one another, and a distal portion in which a second plurality of cells of the expandable frame are at least partially separated from one another.

## Description

### [TECHNICAL FIELD]

The present disclosure relates to a thrombectomy device, and more particularly, but not limited thereto, to a mechanical thrombectomy device used for removing a thrombus from a blood vessel.

### [BACKGROUND]

Various methods exist for treating a thrombus formed at a particular blood vessel site of a body. Among them, a mechanical thrombectomy device is inserted into a blood vessel to break up or remove the thrombus within the blood vessel and is used to restore perfusion through an occluded blood vessel. Mechanical thrombectomy devices approved for such purposes include thrombus filters, thrombus aspiration devices, coil retrievers, and more recently, stent retriever devices.

A stent retriever device is primarily used to remove an acute thrombus from a stroke patient. A type of self-expanding stent formed of a wire mesh is deployed in a blood vessel to capture the thrombus, and the stent is then retrieved from the patient together with the thrombus by pulling a support wire of the stent retriever. In terms of usability and performance, such a stent retriever device is required to provide visibility during fluoroscopy, to capture, restrain, or retain a thrombus without damaging a blood vessel in a narrow and tortuous vascular structure, and to avoid creating dangerous situations during a procedure.

A conventional stent retriever device having a cylindrical structure in which all cells of the stent are directly connected has a problem in that, when inserted into a narrow blood vessel and an expandable frame structure may no longer be compressed, a radial force of the expandable frame structure increases rapidly, thereby causing undesired damage to vascular tissue.

In addition, a conventional stent retriever device having a cylindrical structure in which all cells of the stent are open has a safety problem in that, when the stent retriever device moves through a narrow and tortuous blood vessel, the thrombectomy device is stretched more than necessary, resulting in loss of a captured thrombus, or fatigue of the device increases due to repeated excessive tensile deformation.

### [SUMMARY]

A radial profile of a conventional stent retriever device is uniformly maintained throughout the device and cannot be adjusted. As a result, there are problems in that delicate tissue, such as a narrow blood vessel, may be damaged, and durability of the device may be reduced due to excessive repeated stretching.

An object of the present disclosure for solving the above-described problems is to provide a mechanical thrombectomy device having a distal portion in which cells of the expandable frame of the clot arrestor are at least partially separated from one another, such that a radial profile of a stent retriever device can be adjusted.

Another object of the present disclosure for solving the above-described problems is to provide a mechanical thrombectomy device in which an expandable frame of a clot arrestor has an interconnected proximal portion while allowing adjustment of a radial profile, such that transmission of a pushing force or a pulling force of a stent retriever device is excellent and axial elongation can also be controlled.

However, the technical problems to be solved by the present disclosure are not limited thereto, and may be variously expanded without departing from the spirit and scope of the present disclosure.

A thrombectomy device according to one embodiment of the present disclosure for achieving the above-described objects is a mechanical thrombectomy device including: a support wire; and a clot arrestor connected to the support wire and including an expandable frame, in which the expandable frame may include a proximal portion in which a first plurality of cells of the expandable frame are connected to one another, and a distal portion in which a second plurality of cells of the expandable frame are at least partially separated from one another.

According to one aspect, the first plurality of cells may be connected by connection bridges interconnecting respective cells, and the second plurality of cells may be configured such that each separated cell of the distal portion forms a branch bridge.

According to one aspect, the expandable frame of the proximal portion may include a ring of frame cells, and the ring of frame cells may be formed by a support strut that is a part of the interconnected first plurality of cells, and one end of the support strut may be attached to the support wire, and the other end thereof may be connected to the connection bridge to mechanically support the expandable frame.

According to one aspect, when the clot arrestor is moved through a curved blood vessel, an amount of tensile deformation of the first plurality of cells may be smaller than an amount of tensile deformation of the second plurality of cells.

According to one aspect, the distal portion may have an adjustable radial profile.

According to one aspect, the distal portion may be deformable such that, when the distal portion is deployed in a free space, a radial plane of the distal portion is wider than a radial plane of the proximal portion, and when the distal portion is introduced into a blood vessel, the radial plane of the distal portion is narrower than the radial plane of the proximal portion.

According to one aspect, the branch bridge may branch in a longitudinal direction of the expandable frame.

According to one aspect, the branch bridge may branch in a radial direction of the expandable frame.

According to one aspect, a branch line connecting branch points of the branch bridge may have a linear shape in a longitudinal direction along the expandable frame.

According to one aspect, the branch line may include a plurality of straight lines having different lengths.

According to one aspect, at least two straight lines among the plurality of straight lines may be arranged in directions symmetrical to each other with respect to a longitudinal axis.

According to one aspect, the mechanical thrombectomy device may further include a branch line that branches at least partially in a lateral direction from a longitudinal direction of the expandable frame.

According to one aspect, branch portions of the branch bridge may at least partially overlap one another.

According to one aspect, the branch bridges may be at least partially spaced apart from one another in a longitudinal direction or a radial direction with interconnected cells interposed therebetween along the expandable frame of the distal portion.

According to one aspect, a branch line connecting branch points of the branch bridge may have a spiral shape along a circumference of the expandable frame of the distal portion.

According to one aspect, a branch line connecting branch points of the branch bridge may be wavy in a longitudinal direction along the expandable frame of the distal portion.

According to one aspect, the branch bridge may be ground or tapered to form a smooth edge.

According to one aspect, a longitudinal length of the proximal portion and a longitudinal length of the distal portion may be different from each other.

According to one aspect, a ratio between the longitudinal length of the proximal portion and the longitudinal length of the distal portion may be differently determined depending on a target blood vessel into which the mechanical thrombectomy device is to be inserted.

According to one aspect, the expandable frame may be formed by laser-cutting a shape memory alloy tube.

The disclosed technology may provide the following effects. However, this does not mean that a particular embodiment must include all of the following effects or only the following effects. Accordingly, the scope of the disclosed technology should not be construed as being limited thereby.

According to the mechanical thrombectomy device according to one embodiment of the present disclosure described above, due to the clot arrestor including the interconnected proximal portion and the at least partially separated distal portion, the radial force of the mechanical thrombectomy device may partially vary. Accordingly, a mechanical thrombectomy device may be provided that causes less damage to delicate blood vessels while providing excellent structural support.

In addition, the mechanical thrombectomy device according to one embodiment of the present disclosure described above allows a radial profile of the mechanical thrombectomy device to be adjusted differently in part, thereby enabling deep access into complex and narrow blood vessels, preventing excessive tensile deformation, and providing stable and excellent thrombus removal performance.

### [BRIEF DESCRIPTION OF THE DRAWINGS]

FIG. 1 is a plan view of a mechanical thrombectomy device according to one embodiment of the present disclosure.
FIG. 2 is a plan view of a clot arrestor according to one embodiment of the present disclosure.
FIG. 3 is an enlarged view of a connection bridge according to one embodiment of the present disclosure.
FIG. 4 is an enlarged view of a branch bridge according to one embodiment of the present disclosure.
FIG. 5 is a view partially illustrating a proximal portion of the clot arrestor according to one embodiment of the present disclosure.
FIG. 6 is a view for explaining a branch bridge according to one embodiment of the present disclosure.
FIG. 7 is a view for explaining a branch bridge according to one embodiment of the present disclosure.
FIG. 8 is a view for explaining an arrangement of branch bridges according to one embodiment of the present disclosure.
FIG. 9 is a view for explaining another arrangement of branch bridges according to one embodiment of the present disclosure.
FIG. 10 is a view for explaining still another arrangement of branch bridges according to one embodiment of the present disclosure.
FIG. 11 is a view for explaining yet another arrangement of branch bridges according to one embodiment of the present disclosure.
FIG. 12 is a view for explaining yet another arrangement of branch bridges according to one embodiment of the present disclosure.
FIG. 13 is a view for explaining yet another arrangement of branch bridges according to one embodiment of the present disclosure.
FIG. 14 is a view illustrating a shape of a branch bridge and a radial profile of a distal portion corresponding thereto according to one embodiment of the present disclosure.
FIG. 15 is a view illustrating another shape of a branch bridge and a radial profile of a distal portion corresponding thereto according to one embodiment of the present disclosure.
FIG. 16 is a view illustrating still another shape of a branch bridge and a radial profile of a distal portion corresponding thereto according to one embodiment of the present disclosure.

### [DETAILED DESCRIPTION]

The present disclosure may be modified in various ways and may have various embodiments, and specific embodiments will be illustrated in the drawings and described in detail.

However, this is not intended to limit the present disclosure to particular embodiments, and it should be understood that all modifications, equivalents, and substitutes falling within the spirit and technical scope of the present disclosure are included therein.

Terms such as first and second may be used to describe various elements, but the elements should not be limited by these terms. These terms are used only for the purpose of distinguishing one element from another element. For example, a first element may be referred to as a second element without departing from the scope of the present disclosure, and similarly, a second element may be referred to as a first element. The term "and/or" includes any combination of a plurality of related listed items or any one of the plurality of related listed items.

When an element is referred to as being "connected" or "coupled" to another element, it should be understood that it may be directly connected or coupled to the other element, or intervening elements may be present therebetween. In contrast, when an element is referred to as being "directly connected" or "directly coupled" to another element, it should be understood that no intervening element is present therebetween.

The terminology used herein is for the purpose of describing particular embodiments only and is not intended to limit the present disclosure. Singular forms include plural forms unless the context clearly indicates otherwise. As used herein, the terms "include" or "have" are intended to specify the presence of stated features, numbers, steps, operations, elements, components, parts, or combinations thereof, and should not be construed as precluding the presence or addition of one or more other features, numbers, steps, operations, elements, components, parts, or combinations thereof.

Unless otherwise defined, all terms used herein, including technical and scientific terms, have the same meanings as commonly understood by a person having ordinary skill in the art to which the present disclosure pertains. Terms defined in commonly used dictionaries should be interpreted as having meanings consistent with their meanings in the context of the relevant art and should not be interpreted in an idealized or overly formal sense unless expressly defined herein.

Hereinafter, preferred embodiments of the present disclosure will be described in greater detail with reference to the accompanying drawings. In describing the present disclosure, the same reference numerals are used to designate the same elements throughout the drawings in order to facilitate overall understanding of the present disclosure, and redundant descriptions of the same elements will be omitted.

As discussed above, conventional stent retriever devices had structures in which all cells of a stent were either directly connected to one another or entirely separated from one another. Accordingly, a radial force of an expandable frame of a clot arrestor could not be made partially different. As a result, when the radial force abruptly increases, there was a problem in that damage to vascular tissue may be caused. Conversely, when the radial force is too small, the clot arrestor may be excessively elongated, and thus may not effectively remove a thrombus, and there was a problem with durability of the device due to repeated excessive tensile deformation.

A mechanical thrombectomy device according to one embodiment of the present disclosure is intended to solve the above-described problems. The proximal portion of the clot arrestor is formed of interconnected cells to provide structural support against repeated tensile deformation, thereby being able to improve durability of the mechanical thrombectomy device. In addition, the distal portion of the clot arrestor is formed of partially separated cells to prevent abrupt changes in radial force, thereby being able to provide a mechanical thrombectomy device capable of preventing damage to delicate vascular tissue.

Hereinafter, a mechanical thrombectomy device according to one embodiment of the present disclosure will be described in greater detail with reference to the drawings.

Hereinafter, in the present disclosure, a "clot arrestor" may refer, for example, to a portion of a general stent retriever in which a stent is included. However, the term "clot arrestor" is not limited only to a conventional stent retriever and should be understood as including all components required to restore perfusion by removing coagulated blood from a blood vessel.

Further, hereinafter, in the present disclosure, an "expandable frame" refers to a frame that is initially maintained in a compressed state and may be expanded after being moved to an appropriate location, and it should be understood that any form known in the art may be applied thereto.

In addition, the use of relative terms throughout the Description of the Invention of the present specification may indicate relative positions or directions. For example, "distal" may indicate a first direction along a longitudinal axis of a support wire or a clot arrestor. Likewise, "proximal" may indicate a second direction opposite to the first direction. However, these terms are provided to establish relative references and are not intended to limit the use or orientation of the mechanical thrombectomy device to specific configurations described in the various embodiments below.

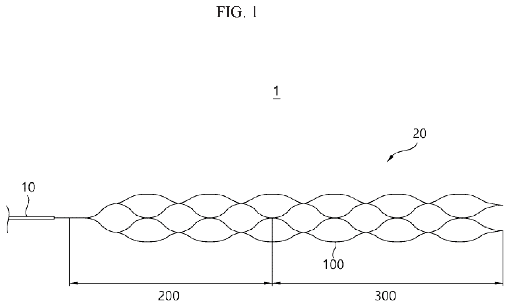

FIG. 1 is a plan view of the mechanical thrombectomy device 1 according to one embodiment of the present disclosure. As illustrated in FIG. 1, the mechanical thrombectomy device 1 according to one embodiment may include, for example, a support wire 10 and a clot arrestor 20.

The mechanical thrombectomy device 1 is an endovascular tool that may be used to treat acute ischemic stroke. The mechanical thrombectomy device 1 includes a proximal control region that is used by an operator to advance, retrieve, and rotate a distal operating region of the device. More specifically, the mechanical thrombectomy device 1 includes a support wire 10 that may be pushed by the operator to advance the distal operating region, pulled by the operator to retrieve the distal operating region, or rotated by the operator to rotate the distal operating region. In one embodiment, the support wire 10 may be a flexible elongated wire formed of a resilient material such as stainless steel or a super-elastic nickel titanium alloy.

The mechanical thrombectomy device 1 may include the clot arrestor 20 that may be advanced through a microcatheter and deployed from the microcatheter to a target tissue. When deployed within the target tissue, the clot arrestor 20 may capture, entrap, restrain, or mechanically integrate with a thrombus. The restrained thrombus may then be retrieved from a patient by pulling the support wire 10 to retrieve the clot arrestor 20 and the thrombus from a vascular structure.

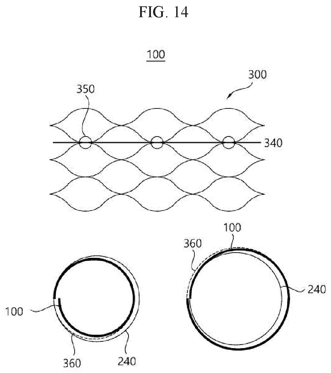

The clot arrestor 20 may be sized and shaped to provide respective degrees of thrombus restraint, thrombus capture, flexibility, or any other performance characteristic. The clot arrestor 20 may be connected to a distal end of the support wire 10 and may include an expandable frame 100. The expandable frame 100 may have a generally elongated cylindrical frame structure capable of capturing and restraining a thrombus within a cylindrical interior thereof, and may be manufactured from a shape memory alloy material including a nickel titanium alloy having flexibility, shape memory, and contraction characteristics. The size and shape of each cell of the expandable frame 100 may be determined according to the above-described performance characteristics of the thrombectomy device. The expandable frame 100 of the clot arrestor 20 may be formed by laser-cutting a cylindrical shape-memory alloy tube into a three-dimensional expandable structure, or by machining, chemical machining, electromechanical machining, electrical discharge machining, or various methods known in the art. Accordingly, the expandable frame 100 of the clot arrestor 20 may have a substantially circular radial profile, as illustrated in FIGS. 14 to 16, which will be described later.

Although not illustrated in the drawings, a plurality of radiopaque markers may be mounted on the expandable frame 100. The radiopaque markers may increase visibility of the device and provide a clue that allows an operator to recognize a position of a thrombus with respect to the clot arrestor. The radiopaque markers may include radiopaque markers mounted by various methods including radiopaque bands, wire coils, plating, welding, coating, or the like, which are commonly used in the art. Alternatively, a radiopaque material may be plated on the expandable frame 100. Alternatively, the radiopaque markers may be formed as bands crimped onto the expandable frame 100. In one aspect, the radiopaque markers may include coils formed of radiopaque wires and may be wound or wrapped around the expandable frame 100. In one aspect, the radiopaque markers may have an atraumatic surface that does not damage a blood vessel wall during use.

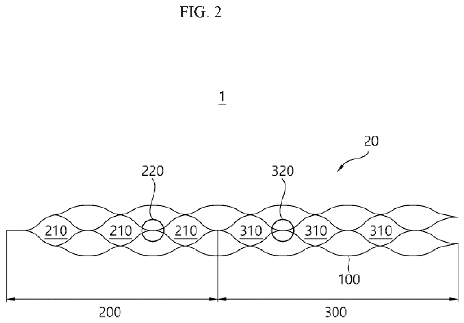

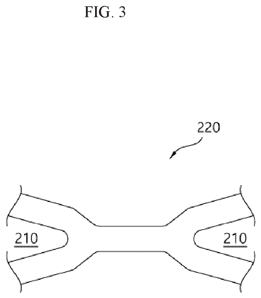

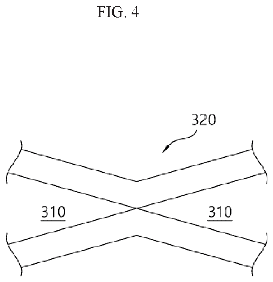

FIG. 2 is a plan view of the clot arrestor 20 according to one embodiment of the present disclosure, FIG. 3 is an enlarged view of a connection bridge 220 according to one embodiment of the present disclosure, and FIG. 4 is an enlarged view of a branch bridge 320 according to one embodiment of the present disclosure.

First, the expandable frame 100 will be described with reference to FIG. 2. The expandable frame 100 includes a proximal portion 200 in which a first plurality of cells 210 of the expandable frame are connected to one another, and a distal portion 300 in which a second plurality of cells 310 of the expandable frame 100 are at least partially separated from one another. Although the respective cells 210 and 310 are illustrated as having a pattern including a generally sinusoidal shape, the pattern of the cells 210 and 310 is not limited thereto, and any form according to performance characteristics of the clot arrestor 20 may be possible.

The proximal portion 200 may refer to a portion starting from a proximal most end of the clot arrestor 20 connected to the support wire 10 illustrated in FIG. 1 to a point at which the first plurality of cells 210 of the expandable frame 100 are connected to one another in the longitudinal direction of the expandable frame 100. In addition, the distal portion 300 may refer to a portion starting from a point at which the proximal portion 200 ends to a distalmost end of the clot arrestor 20, in which the second plurality of cells 310 are at least partially separated from one another in the longitudinal direction of the expandable frame 100.

The first plurality of cells 210 of the proximal portion 200 are connected by the connection bridges 220 interconnecting the respective cells 210, and each of the separated cells 310 of the second plurality of cells 310 of the distal portion 300 may form the branch bridge 320. The connection bridge 220 and the branch bridge 320 are illustrated in enlarged views in FIGS. 3 and 4. Referring to FIG. 3, the connection bridge 220 according to one aspect may be a linear strut connected to each end of adjacent cells of the first plurality of cells 210, but is not limited thereto, and any connection structure may be included in the connection bridge. Referring to FIG. 4, in the branch bridge 320, struts forming adjacent cells of the second plurality of cells 310 may contact one another but are not directly connected to one another. Any form in which the struts of the second plurality of cells 310 do not contact one another may be included in the branch bridge 320. The struts forming the second plurality of cells 310 may branch in a form diverging from one another in the longitudinal direction of the expandable frame 100 from a contact point of each cell 310. In one aspect, the branch bridge 320 may be ground or tapered to form a smooth edge. For example, all portions of the branch bridge 320 that may contact a blood vessel surface may be rounded using any known methods so as to form an atraumatic surface that does not damage the blood vessel surface. The shape and arrangement of the branch bridge 320 will be described in detail below.

Although the above-described proximal portion 200 and distal portion 300 are illustrated in FIGS. 1 and 2 as having generally similar longitudinal lengths, in one aspect, the longitudinal lengths of the proximal portion 200 and the distal portion 300 may be different from each other. In addition, a ratio of the longitudinal lengths of the proximal portion 200 and the distal portion 300 may be differently determined depending on a blood vessel into which the mechanical thrombectomy device 1 is to be inserted.

For example, when the mechanical thrombectomy device 1 needs to be inserted into a relatively narrow and tortuous vascular structure, the longitudinal length of the proximal portion 200 may be formed to be short and the longitudinal length of the distal portion 300 may be formed to be long, thereby adjusting the magnitude of the radial force of the distal portion 300 to be small. Accordingly, the mechanical thrombectomy device 1 may be delivered to a deeper location in a blood vessel while reducing damage to vascular tissue. In addition, when the mechanical thrombectomy device 1 needs to be inserted into a relatively wide blood vessel having an uncomplicated structure, the longitudinal length of the proximal portion 200 may be formed to be longer than the longitudinal length of the distal portion 300, thereby increasing the overall structural coupling force and support force of the mechanical thrombectomy device 1. Accordingly, a thrombus in a blood vessel may be more reliably captured and retrieved.

More specifically, advantages and disadvantages of a frame having a closed cell structure in which all of the cells 210 of the expandable frame 100 are connected to one another, like the proximal portion 200 according to one embodiment of the present disclosure, will first be described.

Advantages of the closed cell structure are as follows: 1. strong mechanical support force can be provided due to structural connections between closed cells, thereby supporting an inner wall of a blood vessel and maintaining stability; and 2. since the closed cell structure is structurally connected, it is easy to accurately deploy the closed cell structure in a blood vessel, and tensile deformation is small during movement in the blood vessel, making the closed cell structure effective for thrombus capture and retrieval.

Meanwhile, disadvantages of the closed cell structure are as follows: 1. flexibility may be reduced due to strong structural connections in the closed cell structure, and this may limit the ability of the clot arrestor to move naturally according to movement of a blood vessel; 2. it may be difficult to distribute radial force due to structural connections between the respective cells in the closed cell structure, and as a result, radial force may sharply increase at a specific point, causing damage to a blood vessel wall; and 3. when a blood vessel has special vascular anatomical characteristics such as a bending portion or a branch portion of the blood vessel, the closed cell structure may have limited adaptability depending on the structure of the blood vessel.

Now, advantages and disadvantages of a frame having an open cell structure in which the cells 310 of the expandable frame 100 are at least partially separated from one another, like the distal portion 300 according to one embodiment of the present disclosure, will be described. The frame having the open cell structure may have aspects that conflict with those of the frame having the closed cell structure.

Advantages of the open cell structure are as follows: 1. the open cell structure can improve flexibility and pliability of the clot arrestor and can help the clot arrestor bend according to pressure and move according to natural movement of a blood vessel; and 2. the open cell structure can distribute pressure between the clot arrestor and a blood vessel wall and reduce radial force applied to the blood vessel wall, thereby providing effective stability without damaging the blood vessel wall.

Disadvantages of the open cell structure are as follows: 1. when a thrombus is located in an opening or slit, there is a risk that the thrombus cannot be effectively removed, which may reduce blood fluidity; 2. the open cell structure may have structural limitations in the size and shape of the opening or slit, which may make it difficult to apply to specific vascular anatomy; and 3. the open cell structure may undergo excessive tensile deformation due to the presence of the opening or slit, which may result in loss of a captured thrombus or make it difficult to capture and remove a thrombus.

When an operator actually uses, during a procedure, a thrombectomy device composed only of the above-described closed cell structure or a thrombectomy device composed only of the open cell structure, each of these structures exhibits a similar level in terms of procedure success rate or mortality due to the respective disadvantages of these structures. Accordingly, the mechanical thrombectomy device 1 according to one embodiment of the present disclosure may be configured such that, in order to complement the advantages and disadvantages of the closed cell structure and the open cell structure, the expandable frame 100 includes the proximal portion 200 having the first plurality of cells 210 connected to one another and the distal portion 300 having the second plurality of cells 310 at least partially separated from one another. That is, according to one embodiment of the present disclosure, the mechanical thrombectomy device 1 including the proximal portion having a closed cell structure and the distal portion having an at least partially open cell structure may be provided.

The mechanical thrombectomy device 1 including the proximal portion of closed cells and the distal portion of open cells may have the following advantages: 1. by providing the open cell structure at the distal portion of the clot arrestor 20, flexibility and pliability of the clot arrestor 20 can be improved, and since the distal portion 300 of the clot arrestor 20 may bend according to pressure and move according to natural movement of a blood vessel, the mechanical thrombectomy device 1 can be inserted more deeply into a relatively narrow and tortuous blood vessel than a thrombectomy device having only a closed cell structure; 2. the distal portion 300 having the open cell structure can distribute pressure between the clot arrestor 20 and a blood vessel wall and reduce radial force applied to the blood vessel wall, thereby providing effective support force and stability without damaging the blood vessel wall; 3. by providing the closed cell structure at the proximal portion 200 of the clot arrestor 20, strong mechanical support force due to structural connections between closed cells can be provided, thereby preventing excessive elongation caused by an opening or slit of the open cell structure and stably supporting an inner wall of a blood vessel; and 4. by providing the closed cell structure having excellent mechanical coupling force at the proximal portion 200 of the clot arrestor 20, deterioration in durability of the device due to repeated excessive elongation can be compensated for.

However, if the open cell structure is located at the proximal portion 200 of the clot arrestor 20 and the closed cell structure is located at the distal portion 300 of the clot arrestor 20, the advantages of the mechanical thrombectomy device 1 according to one embodiment of the present disclosure as described above would not be obtained. Specifically, when the open cell structure is located at the distal portion 300 of the clot arrestor 20, the mechanical thrombectomy device 1 can be inserted more deeply into a relatively narrow and tortuous blood vessel without damaging the blood vessel wall. However, when the closed cell structure is located at the distal portion 300 of the clot arrestor 20, it would be difficult for the radial force of the distal portion 300 to be appropriately distributed, making it difficult to insert the device deep into a blood vessel. If the device is inserted into a portion of a blood vessel having an excessively narrow radius, radial force of the clot arrestor 20 sharply increases, thereby increasing the risk of damaging the blood vessel wall. In addition, when the open cell structure is arranged at the proximal portion 200, the proximal portion 200 of the clot arrestor 20, which is connected to the support wire 10 and transmits a support force for advancing, retrieving, or rotating the mechanical thrombectomy device 1, has low mechanical coupling strength, and thus cannot transmit sufficient support force, making it impossible to use the device stably.

As described above, it may be understood that, as in one embodiment of the present disclosure, locating the open cell structure at the distal portion 300 of the clot arrestor 20 is an important factor in the mechanical thrombectomy device 1, and that the longitudinal lengths of the distal portion 300 and the proximal portion 200 and the ratio of these lengths may be differently determined depending on a blood vessel into which the device is to be inserted, in consideration of the features described above.

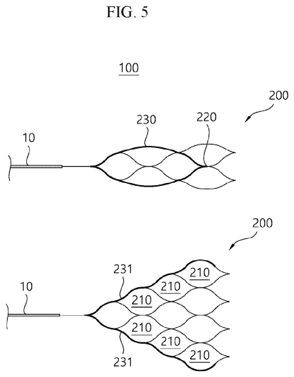

FIG. 5 is a view partially illustrating the proximal portion 200 of the expandable frame 100 according to one embodiment of the present disclosure. The proximal portion 200 of the expandable frame 100 may include a ring 230 of frame cells formed of the cells 210 forming the expandable frame 100. For example, the ring 230 of frame cells may include a plurality of cells 210 connected to one another in a circumferential direction so as to form a cylindrical expandable frame 100 structure. The cell pattern may include one or more struts or bridges and may form an expandable structure having proximal and/or distal openings to restrain a thrombus. The ring 230 of frame cells may also be formed in a ring shape connected as one by support struts 231 that are part of the interconnected first plurality of cells 210. One end of the support strut 231 located proximal to the clot arrestor 20 is attached to the support wire 10, and another end thereof is connected to the connection bridge 220, thereby mechanically supporting the expandable frame 100.

For such mechanical support, in one aspect, the support strut 231 may be formed thicker than struts of other portions forming the expandable frame 100. For example, a thickness of the support strut 231 may be formed to be about 0.025 to 0.04 mm greater than that of struts of other portions of the expandable frame 100, and preferably about 0.028 to 0.038 mm greater. In addition, in the embodiment of FIG. 5, the most proximal end of the proximal portion 200, at which two support struts 231 meet, may be formed thicker than the support struts 231. For example, a thickness of a proximal end of the support strut 231 may be formed to be about 0.01 to 0.03 mm greater than that of the support strut 231, and preferably about 0.012 to 0.025 mm greater. As described above, by partially adjusting the strut thickness of the proximal portion 200 of the expandable frame 100 differently, mechanical support force of the proximal portion 200 can be further increased.

Accordingly, higher mechanical support force and coupling strength may be provided to the first plurality of cells 210 located at the proximal portion 200 of the clot arrestor 20 than to other portions of the clot arrestor 20. As a result, when the clot arrestor 20 is advanced or retrieved through a narrow and tortuous blood vessel, an amount of tensile deformation of the proximal portion 200 may be smaller than an amount of tensile deformation of other portions of the clot arrestor 20. That is, an amount of tensile deformation of the first plurality of cells 210 may be smaller than an amount of tensile deformation of the second plurality of cells 310.

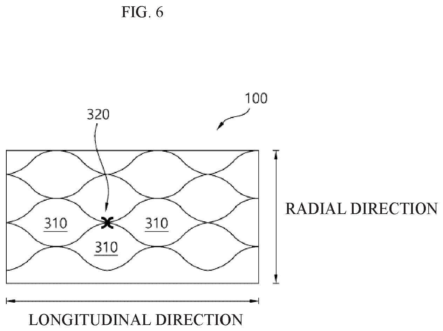

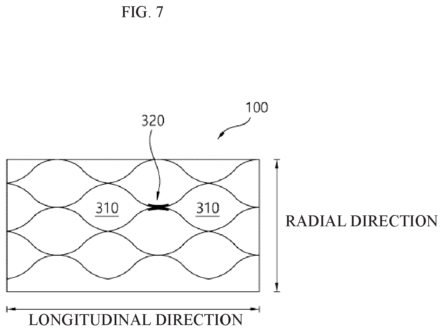

Hereinafter, branching directions of the branch bridge 320 will be described in detail with reference to FIGS. 6 and 7. FIGS. 6 and 7 are views for explaining branch bridges according to one embodiment of the present disclosure. In FIGS. 6 and 7, the branch bridge 320 is highlighted for convenience of description. In the embodiment of FIG. 6, the branch bridge 320 branches in the radial direction of the expandable frame 100, and in the embodiment of FIG. 7, the branch bridge 320 may branch in the longitudinal direction of the expandable frame 100.

As illustrated in FIG. 6, when the branch bridge 320 branches in the radial direction, the second plurality of cells 310 may have an effect as if two cells are connected as one in the radial direction. When the expandable frame 100 is compressed in the longitudinal direction by such a radial branch bridge 320, force inside the expandable frame 100 may be effectively distributed due to generation of an opening by the branch bridge 320. Even when the clot arrestor 20 can no longer be compressed, the clot arrestor 20 may be partially overlapped instead of being folded in the longitudinal direction, thereby securing safety during use of the mechanical thrombectomy device 1.

Meanwhile, as illustrated in FIG. 7, when the branch bridge 320 branches in the longitudinal direction, the second plurality of cells 310 may have an effect as if they form a closed cell structure with one another in the radial direction with the branch bridge 320 therebetween and are connected to one another in the longitudinal direction. When the expandable frame 100 is compressed in the radial direction by such a longitudinal branch bridge 320, radial force inside the expandable frame 100 may be effectively distributed due to generation of an opening caused by the branch bridge 320. Moreover, even when the clot arrestor 20 is pressed in a blood vessel, such as a bending portion or a branch portion of a narrow blood vessel, the distal portion 300 of the clot arrestor 20 in which the branch bridge 320 is formed may be adjusted to have a smaller radial profile according to the vascular structure. The radial profile of the distal portion 300 will be described in detail below with reference to FIGS. 14 to 16.

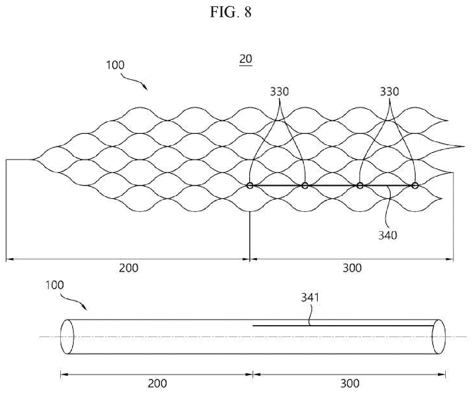

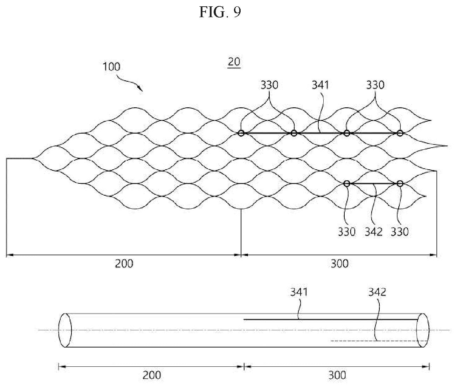

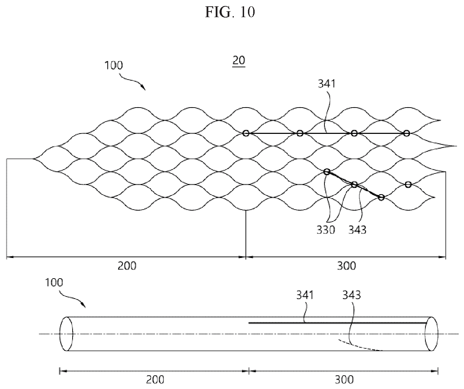

Hereinafter, various arrangements of branch bridges according to one embodiment of the present disclosure will be described with reference to FIGS. 8 to 12. FIG. 8 is a view for explaining an arrangement of branch bridges according to one embodiment of the present disclosure. FIG. 9 is a view for explaining another arrangement of branch bridges according to one embodiment of the present disclosure. FIGS. 10 to 13 are views for explaining still other arrangements of branch bridges according to embodiments of the present disclosure, respectively. For convenience of description, the expandable frame 100 according to one embodiment of the present disclosure is schematically illustrated as a cylindrical shape in FIGS. 8 to 12.

Referring to FIG. 8, the distal portion 300 of the expandable frame 100 may include branches in a linear form schematically illustrated in the drawing. More specifically, when branch points 330 of respective branch bridges formed throughout the distal portion 300 of the expandable frame 100 are connected by a line, a branch line 340 having a shape as illustrated in the expandable frame 100 illustrated in a two-dimensional plan view may be obtained. The branch line 340 may be a first branch line 341 in a linear form extending in the longitudinal direction along the expandable frame 100, as illustrated in the cylindrical expandable frame 100 of FIG. 8. When the distal portion 300 of the expandable frame 100 is viewed from a distal end, a circular circumference forming a radial plane of the distal portion 300 may appear to be interrupted by the first branch line 341. The first branch line 341 may distribute radial force of the distal portion 300 of the expandable frame 100. The radial profile and action of the first branch line 341 will be described below with reference to FIG. 14.

Referring to FIG. 9, in one aspect, the branch lines 341 and 342 may include a plurality of straight lines having different lengths. For example, as illustrated in FIG. 9, when the respective branch points 330 formed in the distal portion 300 of the expandable frame 100 are connected by lines along the longitudinal direction of the distal portion 300, two branch lines 341 and 342 may be obtained in the longitudinal direction of the expandable frame 100. These branch lines may include the linear first branch line 341 formed throughout the distal portion 300 of the expandable frame 100, similar to that illustrated in FIG. 8, and a second branch line 342 having a length different from that of the first branch line 341.

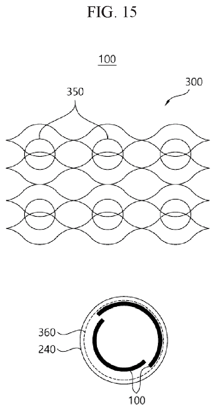

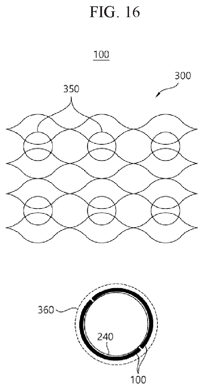

In one aspect, the first branch line 341 and the second branch line 342 may be arranged in directions symmetrical to each other with respect to a longitudinal axis of the expandable frame 100. By arranging the first branch line 341 and the second branch line 342 in symmetrical directions, when the distal portion 300 of the expandable frame 100 is viewed from the distal end, the clot arrestor 20 may appear to be divided into two semicircles. The first branch line 341 and the second branch line 342 may distribute radial force of the distal portion of the expandable frame 100 more than in the embodiment illustrated in FIG. 8, and may also cause the radial profile to be adjusted differently. The radial profiles and actions of the first branch line 341 and the second branch line 342 will be described in detail below with reference to FIGS. 15 and 16.

Referring to FIG. 10, in one aspect, the branch lines 341 and 343 may further include a branch line 343 that branches at least partially in a lateral direction from the longitudinal direction of the expandable frame 100. For example, in the expandable frame 100 illustrated in a two-dimensional plan view, when the respective branch points 330 formed in the distal portion 300 of the expandable frame 100 are connected by lines as illustrated in FIG. 10, the linear first branch line 341 formed throughout the distal portion 300 of the expandable frame 100 may be obtained, as illustrated in the cylindrical expandable frame 100. In addition, when the branch points 330 arranged laterally with respect to the longitudinal axis of the expandable frame 100 are connected by lines, a third branch line 343 extending so as to branch in the lateral direction may be obtained, as illustrated in FIG. 10. The first branch line 341 and the third branch line 343 distribute radial force of the distal portion of the expandable frame 100, thereby allowing the radial profile to be adjusted.

In the embodiment illustrated in FIG. 10, an opening of the distal portion 300 formed by the third branch line 343 does not appear as if the clot arrestor 20 is divided into two portions when viewed from the distal end of the expandable frame 100, unlike the plurality of straight branch lines illustrated in FIG. 9. However, when viewed in the longitudinal direction of the expandable frame 100 illustrated in a cylindrical shape in FIG. 10, the opening may appear as a lateral slit along the circumferential direction of the distal portion 300 of the expandable frame 100, as indicated by the dotted third branch line 343.

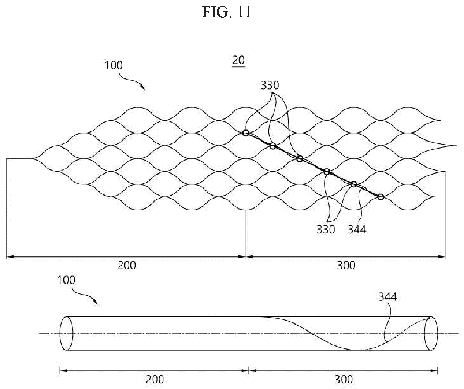

Referring to FIG. 11, in one aspect, a branch line 344 connecting the branch points 330 of the branch bridges 320 may have a spiral shape along the circumference of the expandable frame 100 of the distal portion 300. For example, when the branch points 330 of the respective branch bridges formed throughout the distal portion 300 of the expandable frame 100 are connected by a line, an oblique branch line 344 as illustrated in the two-dimensional plan view of FIG. 11 may be obtained. In the embodiment of FIG. 11, the branch line may include a fourth branch line 344 formed in a spiral shape along the circumference of the expandable frame 100 illustrated in a cylindrical shape. When the distal portion 300 of the expandable frame 100 in which the fourth branch line 344 is formed is viewed from the distal end, a circular circumference forming a radial plane of the distal portion 300 may appear to be interrupted by the fourth branch line 344. The fourth branch line 344 may distribute radial force of the distal portion 300 of the expandable frame 100. The radial profile and action of the fourth branch line 344 will be described below with reference to FIG. 14.

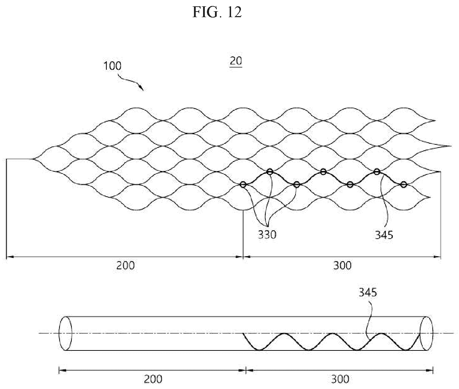

Referring to FIG. 12, in one aspect, a branch line 345 connecting the branch points 330 of the branch bridges 320 may be wavy in the longitudinal direction along the expandable frame 100 of the distal portion 300. For example, when the branch points 330 of the respective branch bridges formed throughout the distal portion 300 of the expandable frame 100 are connected by a line, a wavy branch line 345 as illustrated in the two-dimensional plan view of FIG. 12 may be obtained. In the embodiment of FIG. 12, the branch line 345 may include a fifth branch line 345 having a wavy shape substantially the same as a cell pattern of the distal portion 300 of the expandable frame 100 with respect to a longitudinal axis of the expandable frame 100 illustrated in a cylindrical shape. When the distal portion 300 of the expandable frame 100 in which the fifth branch line 345 is formed is viewed from the distal end, a circular circumference forming a radial plane of the distal portion 300 may appear to be interrupted by the fifth branch line 345. The fifth branch line 345 may distribute radial force of the distal portion 300 of the expandable frame 100. The radial profile and action of the fifth branch line 345 will be described below with reference to FIG. 14.

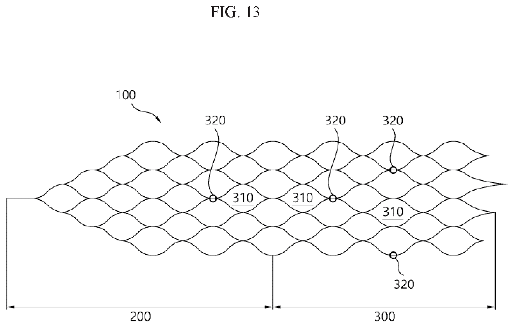

Referring to FIG. 13, in one aspect, the branch bridges 320 may be at least partially spaced apart from one another in the longitudinal direction or the radial direction with interconnected cells 310 interposed therebetween along the expandable frame 100 of the distal portion 300. For example, as illustrated in FIG. 13, the branch points 330 of the respective branch bridges 320 are not connected to one another to form a separate branch line. The branch bridges 320 may be spaced apart from one another in the longitudinal direction of the distal portion 300 of the expandable frame 100. In addition, the branch bridges 320 may be spaced apart from one another in the radial direction of the distal portion 300 of the expandable frame 100. Although not illustrated in the drawings, one of the spaced branch bridges 320 may be continuously arranged with another branch bridge 320 to form a branch line. The above-described arrangement of the branch bridges 320 may form openings in a side surface of the distal portion 300 of the expandable frame 100 to distribute radial force or axial force of the expandable frame 100, and the openings may also serve as inlets through which a thrombus enters and is captured in the expandable frame 100 within a blood vessel.

The shapes of the branch bridges 320 and the respective branch lines 341, 342, 343, 344, and 345 described with reference to FIGS. 8 to 13 may be appropriately selected in consideration of various characteristic conditions, such as a condition of a surgical target and a position, size, and shape of a blood vessel into which the mechanical thrombectomy device 1 is inserted.

Hereinafter, branch portions of the branch bridges 320, and a radial plane 360, a radial profile, and an action of the distal portion 300 of the expandable frame 100 will be described with reference to FIGS. 14 to 16. In FIGS. 14 to 16, for clarity of illustration, the radial profile is illustrated by simplifying and emphasizing a cross-sectional area of the expandable frame 100, and the radial plane of the distal portion 300 is illustrated in an enlarged form with a dotted line around a maximum circumference of the expandable frame 100.

First, the radial profile of the expandable frame 100 will be described. The radial profile refers to a shape of a cross-section cut perpendicular to the longitudinal axis of the expandable frame 100. In general, since the expandable frame 100 has an overall cylindrical shape, the expandable frame 100 may have a circular profile of a constant size over the length thereof. However, since the clot arrestor 20 of the present disclosure has different shapes in the proximal portion 200 and the distal portion 300, the clot arrestor 20 does not have a uniform radial profile as a whole. That is, the proximal portion 200 of the clot arrestor 20 of the present disclosure may have a circular profile, whereas the distal portion 300 may have different radial profiles depending on the shape of the branch bridge 320. For example, when there is one branch line of the branch bridge 320, the radial profile of the distal portion 300 may have a tapered profile in which one end of a cross-section of the distal portion 300 gradually narrows or widens. Alternatively, when a plurality of branch lines of the branch bridge 320 are provided, the cross-section of the distal portion 300 may have a stepped profile formed of a plurality of portions.

Referring to FIG. 14, the distal portion 300 of the expandable frame 100 including the linear branch line 340 as described above with reference to FIG. 8 is partially illustrated. For example, when one linear branch line is provided, in one aspect, a branch portion 350 of the branch bridge 320 may have a form in which branch points contact one another, as illustrated in FIG. 14. In one aspect, when a plurality of linear branch lines are provided, the branch portion 350 of the branch bridge 320 may have a form in which struts of respective cells forming the branch bridge at least partially overlap one another, as illustrated in FIG. 15, or the branch points of the branch bridge 320 may be arranged to be spaced apart from one another without contacting one another, as illustrated in FIG. 16. In one aspect, even when the distal portion 300 has a branch line in the form of one slit that is not straight in the longitudinal direction up to the distal end of the expandable frame 100, as described above with reference to FIGS. 11 and 12, the branch portion 350 of the branch bridge 320 may have a form in which the branch portions contact one another as illustrated in FIG. 14, or may at least partially overlap one another or be spaced apart from one another without contacting one another as illustrated in FIGS. 15 and 16.

In one aspect, the distal portion 300 including the branch portion 350 may be deformable such that, when the distal portion 300 is disposed in a free space in which an external pressure applied to the expandable frame 100 is smaller than a radial force inside the expandable frame 100, a radial plane of the distal portion 300 becomes wider than a radial plane of the proximal portion 200, and when the distal portion 300 is introduced into a blood vessel, the radial plane of the distal portion 300 becomes narrower than the radial plane of the proximal portion 200. For example, in a state in which the branch portions 350 of the distal portion 300 are neither overlapped with nor spaced apart from one another but are arranged in contact with one another according to an initial shape of the cylindrical expandable frame 100, the radial planes of the proximal portion 200 and the distal portion 300 have the same size. However, when the clot arrestor 20 is deployed in a blood vessel and pressed, the distal portion 300 having the branch portion 350 may more effectively distribute radial force than the proximal portion 200, and thus the radial plane 360 of the distal portion 300 may become smaller than the radial plane 240 of the proximal portion 200. In a free space in which the external pressure applied to the expandable frame 100 is smaller than the radial force inside the expandable frame 100, the radial plane 360 of the distal portion 300 may become larger than the radial plane 240 of the proximal portion 200.

Such changes in the radial profile and radial plane 360 of the distal portion 300 are illustrated, for example, in a lower portion of FIG. 14. When the radial plane 360 of the distal portion 300 becomes smaller, the distal portion 300 may have a tapered profile in which a separated end of the expandable frame 100 is rolled inward of the radial plane 360. When the radial plane 360 of the distal portion 300 becomes larger than the radial plane 240 of the proximal portion 200, the distal portion 300 may have a profile in which the separated end of the expandable frame 100 is rolled outward of the radial plane 240 of the proximal portion 200.

Alternatively, since a shape memory alloy tube has flexibility to be deformed into various shapes and an ability to recover a memorized shape, such a change in the radial plane 360 of the distal portion 300 may be enabled by shape-memorizing the distal portion 300 and the proximal portion 200 of the shape memory alloy tube used to manufacture the clot arrestor 20 to have different cross-sectional areas. In addition, the distal portion 300 may be shape-memorized by applying secondary heat treatment and cooling such that, in a free space in which the external pressure applied to the expandable frame 100 is smaller than the radial force inside the expandable frame 100, the radial plane 360 becomes wider than that of the proximal portion 200, and in a narrow blood vessel in which the external pressure applied to the expandable frame 100 is greater than the radial force inside the expandable frame 100, the radial plane 360 becomes narrower than that of the proximal portion 200. Accordingly, during use in a procedure, the distal portion 300 may be inserted into a deep blood vessel while more reliably capturing a thrombus in a relatively wide blood vessel.

Referring to FIG. 15, the distal portion 300 of the expandable frame 100 including two linear branch lines is partially illustrated. For example, when two branch lines are provided, in one aspect, the branch portions 350 may at least partially overlap one another as illustrated in FIG. 15. The radial profile of the distal portion 300 having the overlapping branch portions 350 is as illustrated in a lower portion of FIG. 15, which is emphasized and simplified for explanation. The distal portion 300 of the expandable frame 100 may have a radial profile including two semicircular shapes. The radial profile illustrated in FIG. 15 may be observed when the distal portion 300 of the expandable frame 100 is pressed by receiving radial force. In this case, the radial plane 360 of the distal portion 300 of the expandable frame 100 may become smaller than the radial plane 240 of the proximal portion 200. Due to such a radial profile, the radial force of the distal portion 300 of the clot arrestor 20 may be effectively distributed, so that the distal portion 300 may be delivered to a relatively narrower and deeper blood vessel.

Referring to FIG. 16, the distal portion 300 of the expandable frame 100 including two linear branch lines is partially illustrated. In the embodiment of FIG. 16, the branch portions 350 may be spaced apart from one another without contacting one another. The radial profile of the distal portion 300 is as illustrated in a lower portion of FIG. 16. The distal portion 300 of the expandable frame 100 may have a radial profile including two semicircular shapes, and these semicircular profiles may be arranged to be spaced apart from one another without overlapping one another. The radial profile illustrated in FIG. 16 may be observed when the clot arrestor 20 is disposed in the above-described free space. Due to such a radial profile, the radial force of the distal portion 300 of the clot arrestor 20 may be effectively distributed, thereby effectively removing a thrombus even at a position in a relatively wide blood vessel where it is difficult to deploy a clot arrestor having a closed cell structure.

Although the present disclosure has been described above with reference to the drawings and embodiments, this does not mean that the scope of protection of the present disclosure is limited by the drawings or embodiments. A person skilled in the art will understand that the present disclosure may be variously modified and changed without departing from the spirit and scope of the present disclosure described in the following claims.

Although the present disclosure described above has been described based on a series of functional blocks, the present disclosure is not limited by the above-described embodiments and the accompanying drawings, and it will be apparent to a person having ordinary skill in the art to which the present disclosure pertains that various substitutions, modifications, and changes may be made without departing from the technical spirit of the present disclosure.

Combinations of the above-described embodiments are not limited to the above-described embodiments, and various forms of combinations may be provided according to implementation and/or need, in addition to the above-described embodiments.

In the above-described embodiments, methods have been described based on flowcharts as a series of steps or blocks, but the present disclosure is not limited to the order of the steps, and some steps may occur in an order different from that described above or simultaneously with other steps. In addition, a person having ordinary skill in the art will understand that the steps illustrated in the flowcharts are not exclusive, that other steps may be included, or that one or more steps of the flowcharts may be deleted without affecting the scope of the present disclosure.

The above-described embodiments include examples of various aspects. Although all possible combinations for representing the various aspects cannot be described, a person having ordinary skill in the art will recognize that other combinations are possible. Accordingly, the present disclosure should be understood as including all other substitutions, modifications, and changes falling within the scope of the following claims.

**[DESCRIPTION OF REFERENCE NUMERALS]**

| | | | |
|---|---|---|---|
| 1: | mechanical thrombectomy device | 10: | support wire |
| 20: | clot arrestor | 100: | expandable frame |
| 200: | proximal portion | 210: | first plurality of cells |
| 220: | connection bridge | 230: | ring of frame cells |
| 231: | support strut | 240: | radial plane of proximal portion |
| 300: | distal portion | 310: | second plurality of cells |
| 320: | branch bridge | 330: | branch point |
| 340: | branch line | 341: | first branch line |
| 342: | second branch line | 343: | third branch line |
| 344: | fourth branch line | 345: | fifth branch line |
| 350: | branch portion | 360: | radial plane of distal portion |

## Claims

1. A mechanical thrombectomy device comprising:
a support wire; and
a clot arrestor connected to the support wire and including an expandable frame,
wherein the expandable frame includes
a proximal portion in which a first plurality of cells of the expandable frame are connected to one another, and
a distal portion in which a second plurality of cells of the expandable frame are at least partially separated from one another.

2. The mechanical thrombectomy device of claim 1, wherein the first plurality of cells are connected by connection bridges interconnecting respective cells, and
the second plurality of cells are configured such that each separated cell of the distal portion forms a branch bridge.

3. The mechanical thrombectomy device of claim 2, wherein the expandable frame of the proximal portion includes a ring of frame cells, and
the ring of frame cells is formed by a support strut that is a part of the interconnected first plurality of cells, and one end of the support strut is attached to the support wire, and the other end thereof is connected to the connection bridge to mechanically support the expandable frame.

4. The mechanical thrombectomy device of claim 3, wherein, when the clot arrestor is moved through a curved blood vessel, an amount of tensile deformation of the first plurality of cells is smaller than an amount of tensile deformation of the second plurality of cells.

5. The mechanical thrombectomy device of claim 2, wherein the distal portion has an adjustable radial profile.

6. The mechanical thrombectomy device of claim 5, wherein the distal portion is deformable such that, when the distal portion is deployed in a free space, a radial plane of the distal portion is wider than a radial plane of the proximal portion, and when the distal portion is introduced into a blood vessel, the radial plane of the distal portion is narrower than the radial plane of the proximal portion.

7. The mechanical thrombectomy device of claim 5, wherein the branch bridge branches in a longitudinal direction of the expandable frame.

8. The mechanical thrombectomy device of claim 5, wherein the branch bridge branches in a radial direction of the expandable frame.

9. The mechanical thrombectomy device of claim 5, wherein a branch line connecting branch points of the branch bridge has a linear shape in a longitudinal direction along the expandable frame.

10. The mechanical thrombectomy device of claim 9, wherein the branch line includes a plurality of straight lines having different lengths.

11. The mechanical thrombectomy device of claim 10, wherein at least two straight lines among the plurality of straight lines are arranged in directions symmetrical to each other with respect to a longitudinal axis.

12. The mechanical thrombectomy device of claim 9, further comprising a branch line that branches at least partially in a lateral direction from a longitudinal direction of the expandable frame.

13. The mechanical thrombectomy device of claim 5, wherein branch portions of the branch bridge at least partially overlap one another.

14. The mechanical thrombectomy device of claim 5, wherein the branch bridges are at least partially spaced apart from one another in a longitudinal direction or a radial direction with interconnected cells interposed therebetween along the expandable frame of the distal portion.

15. The mechanical thrombectomy device of claim 5, wherein a branch line connecting branch points of the branch bridge has a spiral shape along a circumference of the expandable frame of the distal portion.

16. The mechanical thrombectomy device of claim 5, wherein a branch line connecting branch points of the branch bridge is wavy in a longitudinal direction along the expandable frame of the distal portion.

17. The mechanical thrombectomy device of claim 2, wherein the branch bridge is ground or tapered to form a smooth edge.

18. The mechanical thrombectomy device of claim 1, wherein a longitudinal length of the proximal portion and a longitudinal length of the distal portion are different from each other.

19. The mechanical thrombectomy device of claim 18, wherein a ratio between the longitudinal length of the proximal portion and the longitudinal length of the distal portion is differently determined depending on a target blood vessel into which the mechanical thrombectomy device is to be inserted.

20. The mechanical thrombectomy device of claim 1, wherein the expandable frame is formed by laser-cutting a shape memory alloy tube.
